Europäisches Patentamt

European Patent Office

Office européen des brevets

(19)

(11) Numéro de publication : **0 475 811 A1**

# (12) DEMANDE DE BREVET EUROPEEN

(21) Numéro de dépôt : **91402334.6**

(51) Int. Cl.$^5$ : **A61F 13/00**

(22) Date de dépôt : **30.08.91**

(30) Priorité : **31.08.90 FR 9010855**

(43) Date de publication de la demande :
**18.03.92 Bulletin 92/12**

(84) Etats contractants désignés :
**AT BE CH DE DK ES GB IT LI LU NL SE**

(71) Demandeur : **ETABLISSEMENTS THUASNE & CIE**
**27, rue de la Jomayère**
**F-42031 St. Etienne Cedex (FR)**
(71) Demandeur : **THUASNE-PARIS**
**6, rue des Marronniers**
**F-92300 Levallois-Perret (FR)**

(72) Inventeur : **Picolet, Jean-Pierre**
**3 Rue des Glycines**
**F-42270 Saint Priest en Jarez (FR)**
Inventeur : **Ducottet, Elisabeth**
**52 Boulevard Vital Bouhot**
**F-92200 Neuilly sur Seine (FR)**
Inventeur : **Courtet, François**
**La Cote, Saint Bonnet les Oules**
**F-43330 Saint Galmier (FR)**

(74) Mandataire : **Coutel, Jean-Claude**
**Cabinet AYMARD & COUTEL 20, rue Vignon**
**F-75009 Paris (FR)**

(54) **Marques de repérage et moyens de corrélation pour la pose de bandages médicaux élastiques.**

(57) Selon l'invention, pour la pose d'un bandage médical présentant une élasticité au moins dans le sens longitudinal, on crée, pour un bandage d'une largeur prédéterminée et connue, une relation entre la traction exercée longitudinalement sur le bandage et une grandeur géométrique locale A′ de la partie du corps recevant le bandage pour que la pression locale P exercée par le bandage sur cette partie du corps soit proportionnelle à ladite traction et inversement proportionnelle à ladite grandeur géométrique.

EP 0 475 811 A1

FIG. 6

La présente invention est relative à la pose de bandages médicaux ou analogues qui présentent des caractéristiques d'élasticité au moins dans le sens longitudinal et qui sont destinés à être enroulés autour d'un membre du corps humain, ou éventuellement animal, dans un but de contention et/ou de soutien.

De tels bandages sont utilisés par exemple en cas d'insuffisance de circulation de retour veineux ou lymphatique, pour lequel il est nécessaire d'exercer une compréssion externe à but prophylactique, thérapeutique, ou de traitement d'entretien.

Le paramètre le plus important qui est à prendre en compte dans l'utilisation de tels bandages élastiques est constitué par la pression locale exercée par le bandage, puisque c'est cette pression qui définit la compression à laquelle le membre en question est soumis. Il va de soi, par analogie avec la posologie des médicaments, qu'une compression trop faible entraîne une action insuffisante de la part du bandage et que, au contraire, une compression trop grande et/ou irrégulière entraîne une action excessive du genre garot. Il est donc essentiel que le bandage soit appliqué de manière à exercer sur la partie du corps concernée une pression prédéterminée appropriée, notamment dégressive.

L'invention a précisément pour but de fournir un dispositif permettant la pose d'un bandage médical élastique pour que celui-ci exerce une pression contrôlée.

A cet effet, le dispositif selon l'invention part de l'idée qui consiste, pour un bandage de largeur prédéterminée et connue, à créer une relation entre la traction longitudinale exercée sur le bandage et une grandeur géométrique locale de la partie du corps recevant le bandage pour que la pression locale exercée par le bandage sur le membre soit proportionnelle à ladite traction et inversement proportionnelle à ladite grandeur géométrique.

Ainsi, en prenant par exemple comme paramètres de base la largeur du bandage et éventuellement le nombre de recouvrements de celui-ci sur lui-même, on détermine, en fonction de la géométrie de la partie du corps à recouvrir, une relation entre la traction à exercer sur le bandage et la pression qui en résulte sur cette partie du corps.

La pression P exercée sur la partie du corps est donnée par la relation $P = k\dfrac{T}{A}$, T étant la traction longitudinale exercée sur le bandage, A étant ladite grandeur géométrique et k étant une constante prenant en compte notamment la largeur du bandage, la nature de la grandeur géométrique considérée, le nombre de recouvrements du bandage sur lui-même et le système d'unités utilisé.

Par exemple, la pression P est donnée par la relation suivante, qui est obtenue à partir de la relation indiquée ci-dessus :

$$P = \frac{1}{L} \times \frac{T}{A} \times n,$$

P étant la pression en Pascals, L étant la largeur de la bande en cm, T étant la traction en Newtons, A étant, en cm, le rayon de courbure de la partie du corps concernée, et n étant le nombre de recouvrements du bandage sur lui-même sur ladite partie du corps.

Cette relation peut également être exprimée d'une autre manière, dans le cas où la partie du corps concernée est considérée comme ayant une section droite circulaire :

$$P = \frac{6,28}{L} \times \frac{T}{A'} \times n,$$

P, L, T et n étant les mêmes que dans la relation précédente et A' étant, en centimètres, le périmètre de la partie du corps concernée.

L'invention vise un dispositif pour la mise en oeuvre du principe ci-dessus. Selon l'invention, ce dispositif est caractérisé par le fait qu'il comporte des repères qui sont portés par le bandage et dont la déformation longitudinale est significative et donne une indication visuelle de la traction longitudinale exercée sur le bandage, et des moyens de corrélation entre ladite pression, ladite traction et ladite grandeur géométrique, avec pour paramètres la largeur du bandage et le nombre de recouvrements de celui-ci sur la partie du corps concernée, ces moyens de corrélation étant agencés pour donner une indication visuelle de la corrélation.

Ces moyens de corrélation peuvent être constitués de diverses manières, par exemple par au moins une famille de graphiques ou par un micro-processeur programmable commandant des moyens d'affichage.

On comprendra bien l'invention à la lecture du complément de description qui va suivre et en référence aux dessins annexés dans lesquels :

Fig. 1 est une vue en perspective d'une partie d'un bandage médical élastique montrant les divers paramètres qui interviennent dans l'action de compression du bandage sur la partie du corps concernée;

Fig. 2 est une vue en plan d'une partie d'un bandage élastique au repos comportant des repères qui permettent de donner une indication visuelle de son degré d'allongement, et par conséquent de la traction longitudinale qu'il subit;

Fig. 3 est une vue analogue à la Fig. 2, le bandage ayant subi un certain allongement ;

Fig. 4 est une vue schématique en perspective montrant le processus de pose d'un bandage élastique sur une jambe ;

Fig. 5 est une vue analogue à la Fig. 4, la pose du bandage étant terminée ;

Fig. 6 est un exemple d'un dispositif à graphiques utilisé dans la mise en oeuvre du principe ci-dessus exposé; et

Fig. 7 est une vue en élévation de face d'un dispositif à calculatrice comportant un micro-processeur programmable et des moyens d'affichage pour la mise en oeuvre du principe ci-dessus exposé.

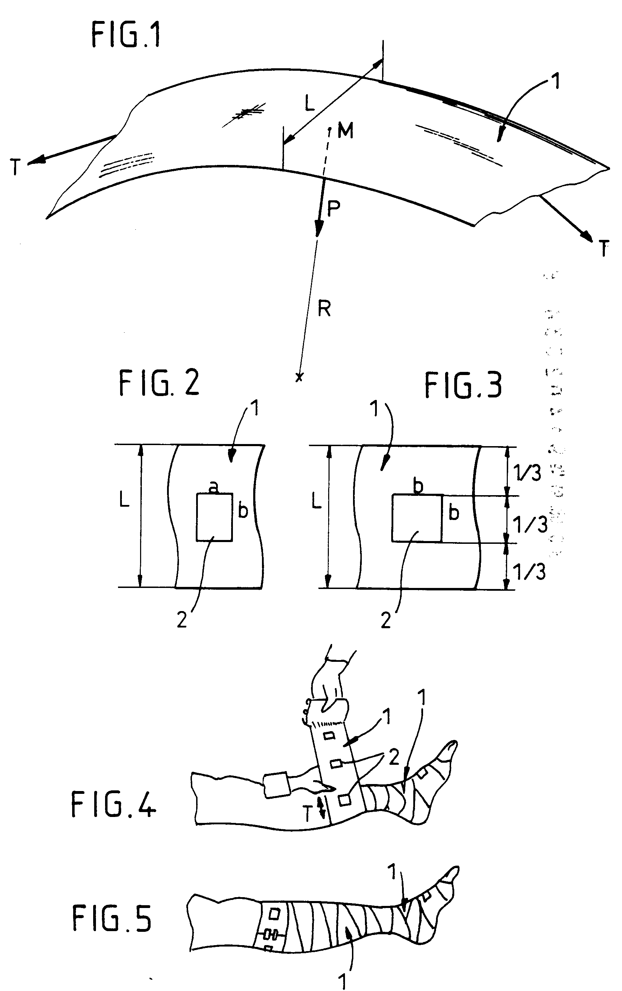

On a représenté en perspective sur la Fig. 1 une partie d'un bandage médical 1 qui présente des caractéristiques d'élasticité au moins dans le sens longitudinal. Ce bandage 1 , d'une largeur L, est destiné à être posé sur une partie du corps, par exemple un membre, pour exercer une action élastique de contention ou de soutien du fait de la pression P qu'il exerce. En tout point M de ladite partie du corps, la pression P exercée par le bandage 1 est proportionnelle à la traction T exercée longitudinalement sur le bandage, inversement proportionnelle à la largeur L du bandage, et elle dépend également de la géométrie locale de ladite partie du corps.

Si la géométrie locale de cette partie du corps est caractérisée par le rayon de courbure R au point M, la pression P exercée par le bandage 1 au point M est donnée par la relation

$$P = k_1 \frac{T}{RL} \,,$$

$k_1$ étant une constante qui dépend du nombre de recouvrements du bandage 1 sur lui-même au point M et du système d'unités utilisé .

Si la géométrie de cette partie du corps est caractérisée par son périmètre, c'est-à-dire si, par approximation, on assimile la section droite de cette partie du corps à un cercle, la pression P est donnée par la relation

$$P = k_2 \frac{T}{A'L} \,,$$

A′ étant le périmètre de cette partie du corps et $k_2$ étant une constante du même type que la constante $k_1$ ci-dessus.

En valeur numérique, les deux relations ci-dessus donnent :

$$P = \frac{1}{L} \times \frac{T}{R} \times n,$$

$$P = \frac{6{,}28}{L} \times \frac{T}{A'} \times n$$

P étant la pression exprimée en Pascals , L étant la largeur du bandage en cm, T étant, en Newtons, la traction longitudinale exercée sur la bandage, R étant, en cm, le rayon de courbure de la partie du corps au point M, n étant le nombre de recouvrements du bandage sur lui-même au point M, et A′ étant , en cm, le périmètre de cette partie du corps.

Pour une partie donnée du corps sur laquelle on désire exercer une pression P prédéterminée, on voit que l'on peut agir sur la traction T, sur la largeur L du bandage et/ou sur le nombre n de recouvrements. Dans la pratique, on ne dispose que d'un certain nombre de largeurs possibles L, de sorte que c'est essentiellement sur T et n que l'on peut agir. Toutefois, il n'est pas possible de donner à T toutes les valeurs théoriquement possibles ; il en résulte que, en général, on agit à la fois sur T et sur n.

On a représenté sur les Figs. 2 et 3 une partie du bandage 1 qui, de manière connue, porte des repères permettant d'apprécier l'allongement du bandage,et par conséquent, suivant les caractéristiques élastiques de celui-ci, la traction T à laquelle il est soumis. Par exemple, ces repères 2 sont constitués, au repos, par des rectangles alignés longitudinalement qui présentent une largeur a et une longueur b, la largeur a étant dans le sens longitudinal du bandage. On peut par exemple choisir les dimensions a et b de telle manière que, pour un allongement de 30%, correspondant à une traction T indiquée par le fabricant du bandage, on obtienne une déformation des repères 2 telle que les rectangles se transforment en des carrés de côté b . On a constaté que l'oeil humain peut apprécier avec une bonne exactitude l'obtention d'un tel carré. Ainsi, l'utilisateur sait que, pour une bande donnée, il exerce une traction T d'une valeur connue lorsque les rectangles sont transformés en des carrés. De tels bandages à repères sont connus par exemple par les documents FR-A-2.499.416 et US-A-3.613.679; mais ces documents n'indiquent nullement des moyens pour qui ces bandages exercent une pression prédéterminée et contrôlée.

Bien entendu, on pourrait adopter tout autre système de repères 2 qui permette, pour un bandage donné, de savoir la traction T qui est exercée en fonction du degré de déformation de ces repères.

Comme montré sur la Fig. 3, la longueur b des rectangles 2, qui est disposée suivant le sens transversal du bandage 1, est égale au tiers de la largeur L de celui-ci et est disposée à égale distance des bords longitudinaux du bandage de telle manière que, lors de la pose, on puisse facilement être guidé par les repères 2 pour réaliser avec précision un recouvrement du bandage sur lui-même à 1/3 ou à 2/3.

On a illustré schématiquement sur les Figs. 4 et 5 le processus de pose du bandage des Figs. 1 à 3. Comme cela sera indiqué ci-après, le bandage 1 est mis manuellement sous la traction T indiquée, avec repérage visuel de la déformation longitudinale des repères 2. Cette pose peut se faire avec recouvrement, comme montré sur les Figs. 4 et 5.

Dans le cas particulier illustré de la jambe, la pression P, pour une traction T de pose constante, va en décroissant de la cheville au mollet, du fait de l'augmentation du périmètre de la jambe, c'est-à-dire de la diminution de son rayon de courbure en section droite, ce qui favorise l'effet médical recherché.

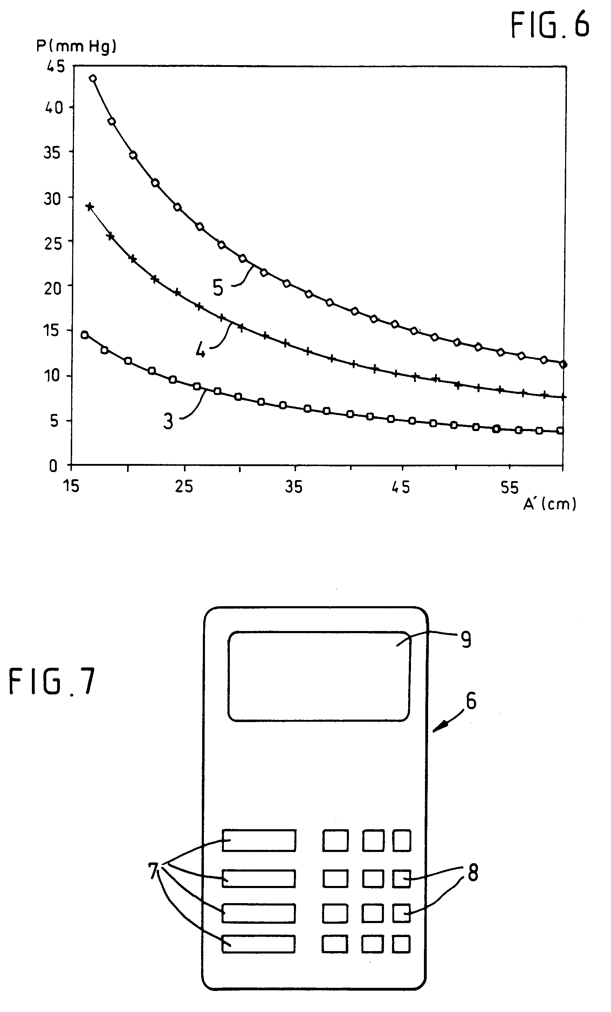

On a illustré sur la Fig. 6 un exemple de moyens visuels de corrélation utilisés pour la mise en oeuvre du principe ci - dessus . Dans cet exemple, on a pris comme paramètres pré-établis la largeur L du bandage et la traction T exercée longitudinalement sur celui-ci, et on a tracé les courbes 3,4 et 5 de la pression exercée en considérant ces paramètres, en fonction du périmètre A′ de la partie du corps considérée.

Les courbes 3,4 et 5 correspondent respectivement à une épaisseur, à deux épaisseurs et à trois épaisseurs de bandage, c'est-à-dire respectivement à zéro recouvrement, un recouvrement et deux recouvrements.

Dans l'exemple de courbes de la Fig. 6, on a considéré le périmètre A' de la partie du corps concernée, car il s'agit là d'une mesure facile à prendre pour le praticien, en tout cas plus facile que le calcul d'un rayon de courbure.

Le praticien, pour un bandage d'une largeur donnée mis sous une tension donnée, connaît avec exactitude la pression locale exercée en fonction du périmètre, la pression P étant la grandeur essentielle à considérer pour le praticien puisqu'elle représente l'effet exercé par le bandage sur la partie du corps concernée, et le périmètre A' étant la grandeur la plus facile à mesurer.

Bien entendu, le praticien peut disposer de plusieurs ensembles de courbes telles que celles de la Fig. 6 selon les bandages qui sont à sa disposition quant à leur largeur et à la traction prédéterminée considérée , c'est-à-dire quant à l'allongement prédéterminé qu'on fait subir aux bandages.

D'une manière générale, les cinq grandeurs qui interviennent dans la pose d'un bandage sont : la largeur L du bandage, la traction T à laquelle il est soumis (ou, ce qui revient au même, son degré d'allongement), le nombre n de recouvrements du bandage sur lui-même , la pression P que ce bandage exerce,et la géométrie de la partie du corps. Parmi ces cinq grandeurs, trois sont considérées comme des paramètres prédéterminés, pouvant néanmoins présenter diverses valeurs, les deux autres étant considérées comme des variables et tracées l'une en fonction de l'autre, comme c'est le cas dans la Fig. 6 où la courbe de pression est tracée en fonction du périmètre A' pour un bandage d'une largeur donnée, soumis à un allongement prédéterminé et pour trois possibilités différentes de recouvrements.

Toutefois, comme représenté sur la Fig. 6, il semble préférable de considérer la largeur, la traction et le nombre de recouvrements comme des paramètres pour obtenir une indication de la pression obtenue en fonction du périmètre local de la partie du corps considérée.

On a représenté schématiquement et partiellement sur la Fig. 7 une variante de réalisation du dispositif selon l'invention. Selon cette variante, le dispositif est constitué par une calculatrice 6 qui contient un microprocesseur programmable (non représenté), des touches de fonctions 7, des touches de chiffres 8 et un écran d'affichage 9 pour la visualisation de la ou des grandeurs de fonctionnement. Par exemple, l'opérateur peut afficher la pression qu'il veut obtenir et le périmètre de la partie du corps concernée, le micro-processeur commandant alors l'affichage 9 pour indiquer la largeur de bande à utiliser, la traction à exercer, notamment par le choix du bandage, et le nombre de recouvrements à exécuter. Bien entendu, il ne s'agit là que d'un exemple d'utilisation. Comme décrit précédemment à propos des graphiques, toutes les variantes utilisables pratiquement sont possibles.

En général et de préférence, on exerce sur tout bandage donné un allongement prédéterminé, par exemple 30 %, qui est atteint lorsque les repères 2 présentent une configuration déformée prédéterminée facilement identifiable par l'oeil humain, cet allongement étant représentatif, avec précision, de la traction T. Il en résulte que, de préférence, pour obtenir une valeur T donnée, on n'agit pas sur l'allongement, mais sur le choix du bandage quant à sa nature et à sa largeur. Le bandage choisi est ensuite allongé de manière prédéterminée propre à ce bandage, le pourcentage d'allongement pouvant être le même ou être différent d'un bandage à l'autre.

## Revendications

1. Dispositif pour la pose d'un bandage médical (1) présentant une élasticité au moins dans le sens longitudinal et une largeur (L) prédéterminée et connue, de manière à créer une relation entre la traction longitudinale (T) exercée sur le bandage (1) et une grandeur géométrique (R,A,A') locale de la partie du corps recevant le bandage (1) pour que la pression locale (P) exercée par le bandage sur cette partie du corps soit proportionnelle à ladite traction (T) et inversement proportionnelle à ladite grandeur géométrique (R,A,A'), caractérisé par le fait qu'il comporte : des repères (2) qui sont portés par le bandage élastique (1) et dont la déformation longitudinale est significative et donne une indication visuelle de la traction longitudinale (T) exercée sur le bandage ; et des moyens (3,4,5,6) de corrélation entre ladite pression, ladite traction et ladite grandeur géométrique, avec pour paramètres la largeur du bandage et le nombre de recouvrements de celui-ci sur ladite partie du corps, ces moyens étant agencés pour donner une indication visuelle de ladite corrélation.

2. Dispositif selon la revendication 1, caractérisé par le fait que les moyens de corrélation sont constitués par des graphiques (3,4,5).

3. Dispositif selon la revendication 1, caractérisé par le fait que les moyens de corrélation sont contenus dans un micro-processeur programmable commandant des moyens d'affichage (9).

4. Dispositif selon l'une des Revendications 1 à 3, caractérisé par le fait que ladite grandeur géométrique est constituée par le rayon de courbure ou le périmètre de ladite partie du corps recevant le bandage.

FIG.1

FIG. 2

FIG.3

FIG.4

FIG.5

7

FIG.6

FIG.7

Office européen **RAPPORT DE RECHERCHE EUROPEENNE**   Numero de la demande
des brevets

EP   91 40 2334

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 499 416 (THUASNE) <br> * page 3, ligne 18 - page 4, ligne 31; figures 1,5-8 * | 1,2,4 | A61F13/00 |
| X | US-A-3 613 679 (BIJOU) <br> * abrégé; figures * <br> * colonne 2, ligne 57 - ligne 67 * | 1 | |
| A | FR-A-2 544 982 (THUASNE) | | |
| A | DE-A-3 640 979 (RAUSCHNER & CO.) | | |

| | DOMAINES TECHNIQUES RECHERCHES (Int. Cl.5) |
|---|---|
| | A61F |

Le présent rapport a été établi pour toutes les revendications

| Lieu de la recherche | Date d'achèvement de la recherche | Examinateur |
|---|---|---|
| LA HAYE | 16 DECEMBRE 1991 | SANCHEZ Y SANCHEZ J. |